(19) Europäisches Patentamt
European Patent Office
Office européen des brevets

(11) Publication number: **0 003 336**
**B1**

(12) **EUROPEAN PATENT SPECIFICATION**

(45) Date of publication of patent specification: **22.02.84**

(21) Application number: **79100163.9**

(22) Date of filing: **19.01.79**

(51) Int. Cl.³: **A 01 N 53/00,**
C 07 C 61/35,
C 07 C 69/743,
C 07 D 209/48,
C 07 D 307/45,
C 07 D 317/54 //C07C69/74,
C07C69/63, C07C67/30,
C07C61/16, C07C51/58,
C07C51/09, C07C121/75,
C07C120/00, C07C67/14,
C07C67/00, C07C67/03,
C07C79/22, C07F9/54,
C07C21/18

(54) **Insecticidal perhaloalkylvinylcyclopropanecarboxylates.**

(30) Priority: **24.07.78 US 927198**
**20.01.78 US 870973**

(43) Date of publication of application:
**08.08.79 Bulletin 79/16**

(45) Publication of the grant of the patent:
**22.02.84 Bulletin 84/8**

(84) Designated Contracting States:
**BE CH DE FR GB LU NL SE**

(56) References cited:
**DE - A - 2 822 472**

(73) Proprietor: **FMC Corporation**
**2000 Market Street**
**Philadelphia Pennsylvania 19103 (US)**

(72) Inventor: **Engel, John Francis**
**802 West Center Street**
**Medina New York 14103 (US)**

(74) Representative: **Vossius Vossius Tauchner**
**Heunemann Rauh**
**Siebertstrasse 4 P.O. Box 86 07 67**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 003 336 B1**

Courier Press, Leamington Spa, England.

Insecticidal perhaloalkylvinylcyclopropanecarboxylates

The present invention is directed to a novel class of cyclopropanecarboxylate insecticides, to a process for preparation, and to an insecticidal method and composition. More particularly, the invention is directed to the insecticidal perhaloalkylvinylcyclopropanecarboxylates set forth in Formula I, their method of manufacture and use.

Pyrethrins, naturally occurring extracts of chrysanthemum flowers, have long been of interest as insecticides. Since elucidation of the structures of these compounds, synthesis efforts have been directed toward preparation of related compounds having enhanced insecticidal activity and improved stability toward air and light. A noteworthy advance in this area was the discovery by Elliott et al. of certain highly active compounds, for example, dihalovinylcyclopropanecarboxylates such as permethrin, the common name for 3-phenoxybenzyl 3-($\beta$,$\beta$-dichlorovinyl)-2,2-dimethylcyclopropanecarboxylate. This class of compounds, set forth in U.S. Patent 4,024,163, issued May 17, 1977, exhibits substantially improved photostability when compared with previously available cyclopropanecarboxylates such as the chrysanthemamates, that is, compounds of formula I in which Y and Z are each methyl.

The prior art cyclopropanecarboxylates such as those described in the foregoing U.S. Patent have shown high levels of activity against insects of the Order Lepidoptera, but many have not shown commercially satisfactory levels of activity against insects of the Order Homoptera, for example, aphids.

Belgian Patent 863151 discloses $\alpha$-substituted or unsubstituted phenoxybenzyl esters of 2,2-dimethyl-3-disubstituted vinylcyclopropanecarboxylic acid in which the $\alpha$-substituent is ethynyl or cyano and the 3-substituents are both haloalkyl groups of 1 to 2 carbon atoms or one is such a haloalkyl group and the other a halogen atom, or a methyl group.

The present invention provides a new class of insecticidal cyclopropanecarboxylates, perhaloalkylvinylcyclopropanecarboxylates, generally exhibiting a high level of insecticidal activity, improved activity against insects of the Order Homoptera, such as aphids, and improved photostability. Thus, the present invention provides new alcohol and acid components to prepare compounds having unexpected levels of scope of activity for the control of various crop pests.

The present invention also provides novel insecticidal compositions of the foregoing compounds, a method of controlling insects, and a process for preparing the compounds.

In this application, the term "lower", as applied to an alkyl group means having 1 to 6 carbon atoms, preferably 1 to 4 carbon atoms. The term "halo" or "halogen" means bromine, chlorine, or fluorine. With respect to a perhaloalkyl group the halogens may be the same or different and are suitably selected from fluorine and chlorine with fluorine being preferred. These definitions are applicable throughout the specification and claims except where a contrary meaning is clearly indicated.

The insecticidal compounds of this invention are cyclopropanecarboxylates of the general formula

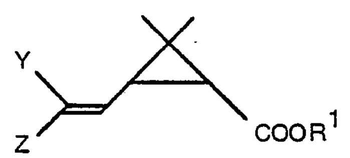

I

wherein one of Y and Z is a perhaloalkyl group having 1 to 4 carbon atoms, preferably 1 to 2 carbon atoms, and the other is hydrogen, halogen, lower alkyl, phenyl, phenylthio, or benzyl, or Y and Z together form a perhalocyclopentylidene group, preferably a perfluorocyclopentylidene group. Particularly desirable compounds are trihalopropenyl, preferably trifluoropropenyl, cyclopropanecarboxylates of formula I in which one of Y and Z is trihalomethyl, preferably trifluoromethyl, and the other is halogen.

$R^1$ represents a wide variety of alcohol residues which form insecticidal esters when combined with chrysanthemic acid [3-(2,2-dimethylvinyl)-2,2-dimethylcyclopropanecarboxylic acid] or the dihalovinylcyclopropanecarboxylic acids of U.S. patent 4,024,163 such as permethrin. $R^1$ thus represents a group useful in the cyclopropanecarboxylate insecticide art to produce insecticidally active esters when combined with an appropriate known pyrethroid acid. For instance $R^1$ may be allethrolonyl, tetrahydrophthalimidomethyl, or may be represented by the formula

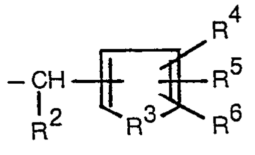

or

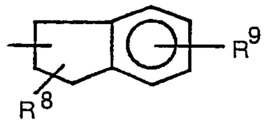

wherein $R^2$ is hydrogen, lower alkyl, ethynyl, cyano, or trihalomethyl; $R^3$ is divalent oxygen, divalent

sulfur, or vinylene; $R^4$, $R^5$ and $R^6$ are independently hydrogen, lower alkyl, halogen, lower alkenyl, phenyl, phenoxy, benzyl, phenylthio, or any two of $R^4$, $R^5$ and $R^6$ are joined to form a divalent methylenedioxy group attached to two adjacent ring carbon atoms of a phenyl ring, and when $R^4$, $R^5$ or $R^6$ contains a phenyl ring that phenyl ring may be substituted with one to three substituents selected from halogen and lower alkyl; $R^8$ is hydrogen, lower alkyl, lower alkoxy, lower alkenyl, lower alkenyloxy, phenyl or benzyl; $R^9$ is hydrogen, halogen, lower alkyl, lower alkoxy, lower alkenyl, lower alkenyloxy, lower alkylthio, lower alkylsulfinyl, lower alkylsulfonyl, phenyl, phenoxy, benzoyl, nitro, or cyano, with the proviso that when one of Y and Z is a perhaloalkyl group, the other of Y and Z is halogen or methyl, $R^3$ is vinylene, and two of $R^4$, $R^5$ and $R^6$ are hydrogen, then

(a) the other of $R^4$, $R^5$ and $R^6$ is not phenoxy, or

(b) $R^2$ is not hydrogen, ethynyl or cyano.

Compounds of formula I containing these alcohol residues in combination with an acid component in which one of Y and Z is trihalomethyl, preferably trifluoromethyl, and the other is halogen exhibit unexpectedly high activity against insects in general, remarkable efficacy against aphids, and, with the exception of 5-benzyl-3-furylmethyl, unusual photo-stability.

The insecticidal compounds and certain intermediates of this invention exist as *cis* and *trans* geometrical isomers; the carboxy and substituted vinyl groups at the 1 and 3 positions of the cyclopropane ring are either *cis* or *trans* with respect to each other. Preparation of such compounds will usually yield a mixture of *cis* and *trans* isomers, designated *cis,trans,* in which the ratio of *cis* to *trans* may vary over a wide range. For purposes of this specification the designations *cis* and *trans* are assigned in accordance with P. E. Burt, et al., *Pestic. Sci., 5* 791—799 (1974). The compounds of this invention may also exist as *E* or *Z* isomers or as mixtures of *E* and *Z* isomers, designated *E,Z,* depending on the spatial relationship of substituents on the $\alpha$-carbon of the vinyl group to those on the $\beta$-carbon of the vinyl group.

In the cyclopropanecarboxylate art it is known there may be substantial differences in the level of insecticidal activity between the *cis* and *trans* isomers. In general, as between the *cis* and *trans* isomer of a given cyclopropanecarboxylate, one isomer is usually more active than the other isomer and also more active than the *cis,trans* mixture. With respect to the present compounds the *cis* isomer is usually the more active. Similar differences in activity may also occur with respect to the *E* and *Z* isomers.

Unless a contrary intent is expressed, the invention embodies and includes all compounds in which the carboxy and substituted vinyl groups at the 1 and 3 positions of the cyclopropane ring are *cis* or *trans,* or a mixture of *cis* and *trans* configuration with respect to each other. Similarly, while the invention is illustrated with a mixture of the *E* and *Z* isomers, the individual isomers, as well as the mixtures, are also contemplated by and within the scope of the present invention. The enantiomers of these isomers are also included within the scope of the invention.

The compounds may be prepared from alkanoates of the formula

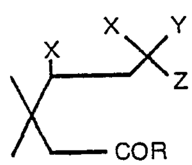

II

in which Y and Z are defined as above; R is lower alkoxy, such as methoxy or ethoxy; and X is chloro or bromo. Example 1 illustrates a method for preparation of intermediates of Formula II by reacting together a lower alkyl 3,3-dimethyl-4-pentenoate and a compound of the formula $X_2$—C(Y)(Z) where X, Y, and Z are as defined above.

This alkanoate is then converted to a compound of the formula

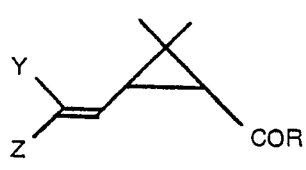

III

in which R is lower alkoxy, hydroxy or halogen and Y and Z are as defined above, for example, by dehydrohalogenating the compound of formula II. This reaction may take place in steps via the intermediates

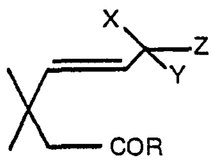

IV

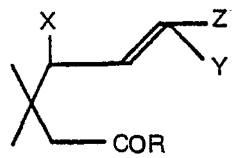

V

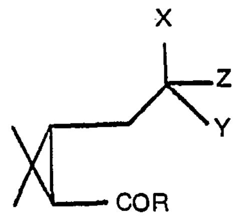

VI

and may be conducted in a single step by removal of 2 halogens or stepwise. These intermediates or mixtures of these may be recovered if desired. The compound of formula III is then converted to the compound of formula I by methods known to the art, for example, by transesterifying with $HOR^1$. Other methods for converting R to $—OR^1$ are well known in the art.

The examples which follow illustrate the use of the method described above for preparation of intermediates and their conversion to insecticidal esters. The conversion of the intermediates illustrates the general method by which claimed insecticidal compounds are prepared. In the examples all temperatures are in degrees centrigrade, all pressures are in mm. Hg, followed, in parenthesis, with the equivalent in pascals or kilopascals, and reduced pressure for concentration of liquid is produced by a water aspirator unless otherwise specified.

Example 1 illustrates the preparation of compounds of formula II.

Example 1

Synthesis of ethyl 3,3-dimethyl-4,6,6-trichloro-7,7,7-trifluoroheptanoate (intermediate)

A stirred solution of 44.6 g (0.267 mole) of ethyl 3,3-dimethyl-4-pentenoate, 100 g (0.533 mole) of 1,1,1-trichlorotrifluoroethane, 0.27 g (0.0027 mole) of cuprous chloride, and 8.2 g (0.134 mole) of ethanolamine in 270 ml of tertiary butyl alcohol, under a nitrogen atmosphere, was heated at reflux for 16 hours. The reaction mixture was cooled to ambient temperature and extracted with three portions of 100 ml each of diethyl ether. A precipitate formed in the extracts was removed by vacuum filtration. The filter cake was washed with two portions of 25 ml each of diethyl ether. The ether extracts were combined with the washings, and the whole was concentrated under reduced pressure to an oily residue. Remaining volatile components were removed from the residue under further reduced pressure using a vacuum pump. The residue was subjected to distillation under reduced pressure to give 78.3 g of ethyl 3,3-dimethyl-4,6,6-trichloro-7,7,7-trifluoroheptanoate; boiling point (b.p.) 85—87° at 0.12—0.15 mm (16—20 Pa). The nmr spectrum was consistent with the assigned structure.

Additional intermediates of formula II, prepared in accordance with the method illustrated in Example 1, are set forth in Table I.

Examples 2 and 3 illustrate preparation of the lower alkyl esters of formula III. Example 2 is a two-step process via the intermediate of formula VI. Example 3 is a one-step process.

Example 2

Synthesis of methyl *cis,trans*-3-[2-chloro-3,3,3-trifluoropropenyl]-2,2-dimethylcyclopropanecarboxylate (intermediate)

A. Preparation of methyl *cis,trans*-3-[2,2-dichloro-3,3,3-trifluoropropyl]-2,2-dimethylcyclopropanecarboxylate as an intermediate

A stirred solution of 37.0 g (0.112 mole) of methyl 3,3-dimethyl-4,6,6-trichloro-7,7,7-trifluoroheptanoate, 50 ml of *tert*-butyl alcohol, 50 ml of dimethylformamide, and 50 ml of hexane, under an argon atmosphere, was cooled to −5°C. To the stirred solution was added dropwise a solution of 16.4 grams (0.14 mole) of potassium *tert*-butoxide in 200 ml *tert*-butyl alcohol at such a rate so as to maintain the reaction mixture temperature at −3 to −5°C. Upon complete addition, the reaction mixture was stirred for 4 hours at −3 to −5°C, then poured into a solution of 8.0 g of ammonium chloride in 250 ml of water. The mixture was extracted with two portions of 200 ml each of diethyl ether. The combined ether extracts were washed with two portions of 200 ml each of water. The ether layer was dried with sodium sulfate and filtered. The filtrate was evaporated under reduced pressure to a residual oil. The oil was distilled under reduced pressure to give 19.8 g of methyl *cis,trans,*-3-[2,2-dichloro-3,3,3-trifluoropropyl]-2,2-dimethylcyclopropanecarboxylate; b.p. 55—57°C/0.09 mm Hg. (12 Pa). The ir and the nmr spectra were consistent with the proposed structure.

Elemental analysis calc'd for $C_{10}H_{13}Cl_2F_3O_2$: C40.98; H4.47
Found: C41.50; H4.41

B. Synthesis of methyl *cis,trans*-3-[2-chloro-3,3,3-trifluoropropenyl]-2,2-dimethylcyclopropanecarboxylate.

A stirred solution of 30.6 g (0.105 mole) of methyl *cis,trans*-3-[2,2-dichloro-3,3,3-trifluoropropyl]-2,2-dimethylcyclopropanecarboxylate and 17.6 g (0.116 mole) of 1,5-diazabicyclo-[5.4.0]undec-5-ene in 100 ml of dimethylformamide was heated at 100°C for 4 hours. The reaction mixture was cooled and poured into a solution of 37.2 ml of concentrated hydrochloric acid in 300 ml of water. The mixture was extracted with three portions of 200 ml each of diethyl ether. The combined ether extracts were washed with an aqueous solution saturated with sodium chloride. The ether layer was dried with sodium sulfate and filtered. The filtrate was evaporated under reduced pressure to give a residual oil. The oil was dissolved in hexane, treated with decolorizing carbon, and filtered. The filtrate was evaporated under reduced pressure to a residual oil. The oil was distilled under reduced pressure to give in three fractions 10.0 g of methyl *cis,trans*-3-[2-chloro-3,3,3-trifluoropropenyl]-2,2-dimethylcyclopropanecarboxylate; b.p. 40—60°C/0.05 mm. (6.7 Pa). The ir and the nmr spectra were consistent with the proposed structure. The nmr spectra indicated an 88:12 mixture of *cis:trans* isomers.

Analyses calc'd for $C_{10}H_{12}ClF_3O_2$: C 46.80; H 4.71;
Found: C 46.91; H 4.79.

Example 3

Synthesis of ethyl *cis,trans*-3-[2-chloro-3,3,3-trifluoropropenyl]-2,2-dimethylcyclopropanecarboxylate (intermediate)

To a stirred solution of 78.3 g (0.228 mole) of ethyl 3,3-dimethyl-4,6,6-trichloro-7,7,7-trifluoroheptanoate in 200 ml of distilled ethanol was added dropwise at ambient temperature 500 ml of an ethanolic solution of sodium ethoxide prepared from 11.5 g of metallic sodium (0.50 mole). After complete addition, the reaction mixture was stirred for one hour at ambient temperature, then allowed to stand for 18 hours. The cloudy reaction mixture was filtered and the filtrate evaporated under reduced pressure to give a residue. The residue was slurried in 200 ml of water, and the mixture was extracted with three portions of 50 ml each of diethyl ether. The combined extracts were dried with sodium sulfate and filtered. The filtrate was evaporated under reduced pressure to give, as a residual oil, 58.5 g of ethyl *cis,trans*-3-[2-chloro-3,3,3-trifluoropropenyl]-2,2-dimethylcyclopropanecarboxylate. The nmr and the ir spectra were consistent with the assigned structure and indicated the product was a mixture of approximately equal parts of *cis* and *trans* isomers.

Additional intermediates of formula VI, prepared in accordance with the method illustrated in Example 2A, are set forth in Table II.

Additional lower alkyl esters of formula III, prepared in accordance with Example 2 or Example 3 above, are set forth as Compounds 3.1 to 3.8 of Table III. Compounds 3.1 through 3.7 were prepared in accordance with Example 2. Compound 3.8 was prepared in accordance with Example 3.

Example 4

Synthesis of *trans* and *cis,trans*-3-[2-chloro-3,3,3-trifluoropropenyl]-2,2-dimethylcyclopropanecarboxylic acid (intermediate)

A solution of 16.2 g (0.06 mole) of ethyl *cis,trans*-3-[2-chloro-3,3,3-trifluoropropenyl]-2,2-dimethylcyclopropanecarboxylate in 94 ml (0.078 mole) of a stock solution containing 3.34 g of sodium hydroxide, 94 ml of ethanol and 6 ml of water was stirred while heating under reflux for a period of 18 hours. The reaction mixture was concentrated under reduced pressure, 25 ml of water was added, and the mixture was acidified to pH 1 using 6N hydrochloric acid. The acidified mixture was extracted with two portions of 50 ml each of diethyl ether. The combined extracts were dried with magnesium sulfate and filtered. The filtrate was evaporated under reduced pressure to give a residue. The residue was heated with 50 ml of hexane. The hot hexane was decanted from a tarry residue and cooled to yield a solid precipitate, which was collected by filtration, then dried to give 3.3 g of solid, m.p. 97—103°C. Concentration of the mother liquor provided a second fraction of solid weighing 0.8 g, m.p. 96—103°C. Nmr spectra of the two fractions indicated the solids were each *trans*-3-[2-chloro-3,3,3-trifluoropropenyl]-2,2-dimethylcyclopropanecarboxylic acid. The mother liquor was evaporated to a residue. The residue was taken up in 50 ml of hexane and the solution cooled in a freezer for 18 hours. A solid precipitate was collected by filtration and dried to give 4.3 g of solid, melting point (m.p.) 64—67°C. An nmr spectrum indicated the solid was a 50/50 mixture of *cis* and *trans* isomers of 3-[2-chloro-3,3,3-trifluoropropenyl]-2,2-dimethylcyclopropanecarboxylic acid.

Additional free acids of formula III, prepared in accordance with Example 4, are set forth as Examples 4.1 through 4.7 of Table III.

Example 5

Synthesis of *trans*-3-[2-chloro-3,3,3-trifluoropropenyl]-2,2-dimethylcyclopropanecarbonyl chloride (intermediate)

To a stirred solution of 4.1 g (0.173 mole) of *trans*-3-[2-chloro-3,3,3-trifluoropropenyl]-2,2-

dimethylcyclopropanecarboxylic acid in 40 ml of toluene at ambient temperature was added 1.7 g (0.022 mole) of pyridine, then 2.6 g (0.022 mole) of thionyl chloride in 25 ml of toluene. Upon complete addition the reaction mixture was stirred at ambient temperature for 17 hours. The reaction mixture was filtered through diatomaceous earth, and the filtrate was evaporated under reduced pressure to give 3.8 g of *trans*-3-[2-chloro-3,3,3-trifluoropropenyl]-2,2-dimethylcyclopropanecarbonyl chloride. The ir spectrum was consistent with the assigned structure.

Additional acid chlorides of formula III, prepared by the method illustrated in Example 5, are set forth as Examples 5.1 through 5.8 in Table III.

Examples 6 through 10 demonstrate the general methods for preparation of insecticidal esters from the foregoing intermediates, and also illustrate the method by which the claimed insecticidal esters may be prepared.

Example 6

Synthesis of 3-phenoxybenzyl *trans*-3-[2-chloro-3,3,3-trifluoropropenyl]-2,2-dimethylcyclopropanecarboxylate

To a stirred solution of 1.8 g (0.007 mole) of *trans*-3-[2-chloro-3,3,3-trifluoropropenyl]-2,2-dimethylcyclopropanecarbonyl chloride in 10 ml of methylene chloride at ambient temperature was added a solution of 1.6 g (0.008 mole) of 3-phenoxybenzyl alcohol and 0.73 g (0.009 mole) of pyridine in 5 ml of methylene chloride. Upon complete addition the reaction mixture was stirred at ambient temperature for 3 hours, then poured into 50 ml of water. The organic layer was separated, and the aqueous layer was extracted with three portions of 50 ml each of methylene chloride. The combined extracts were dried with sodium sulfate, filtered, and the filtrate evaporated under reduced pressure to a residual oil. Volatile components were removed from the oil at 125°C/0.05 mm (6.7 Pa) using a Kugelrohr distillation system. The pot residue was determined 99% 3-phenoxybenzyl *trans*-3-[2-chloro-3,3,3-trifluoropropenyl]-2,2-dimethylcyclopropanecarboxylate, as determined by gas chromatographic analysis. The weight of product was 2.0 g. The nmr and ir spectra were consistent with the assigned structure.

Analysis calc'd for $C_{22}H_{20}ClF_3O_3$: C 62.05 H 4.73
Found: C 62.29 H 4.80

Example 7

Synthesis of $\alpha$-cyano-3-phenoxybenzyl *trans*-3-[2-chloro-3,3,3-trifluoropropenyl]-2,2-dimethylcyclopropanecarboxylate

This compound was prepared in the manner of Example 6 using 1.8 g (0.007 mole) of *trans*-3-[2-chloro-3,3,3-trifluoropropenyl]-2,2-dimethylcyclopropanecarbonyl chloride, as prepared in Example 4, 1.7 g (0.008 mole) of $\alpha$-cyano-3-phenoxybenzyl alcohol, and 0.73 g (0.009 mole) of pyridine in 15 ml of methylene chloride. The pot residue analyzed 98.9% $\alpha$-cyano-3-phenoxybenzyl *trans*-3-[2-chloro-3,3,3-trifluoropropenyl]-2,2-dimethylcyclopropanecarboxylate, as determined by gas chromatographic analysis. The weight of product was 2.4 g. The nmr and the ir spectra were consistent with the assigned structure.

Analysis calc'd for $C_{23}H_{19}ClF_3NO_3$: C 61.27 H 4.25
Found: C 61.57 H 4.38

Example 8

Synthesis of 3-phenoxybenzyl *cis,trans*-3-[2-chloro-3,3,3-trifluoropropenyl]-2,2-dimethylcyclopropanecarboxylate

This compound was prepared in the manner of Example 6 using 1.8 g (0.006 mole) of *cis,trans*-3-[2-chloro-3,3,3-trifluoropropenyl]-2,2-dimethylcyclopropanecarbonyl chloride from Example 4, 1.4 g (0.007 mole) of 3-phenoxybenzyl alcohol, and 0.66 g (0.008 mole) of pyridine in 15 ml of methylene chloride. The pot residue analyzed 99% 3-phenoxybenzyl *cis,trans*-3-[2-chloro-3,3,3-trifluoropropenyl]-2,2-dimethylcyclopropanecarboxylate, as determined by gas chromatographic analysis. The weight of product was 1.0 g. The nmr and ir spectra were consistent with the assigned structure.

Analysis calc'd for $C_{22}H_{20}ClF_3O_3$: C 62.01 H 4.49
Found: C 62.11 H 4.58

Example 9

Synthesis of $\alpha$-cyano-3-phenoxybenzyl *cis,trans*-3-[2-chloro-3,3,3-trifluoropropenyl]-2,2-dimethylcyclopropanecarboxylate

This compound was prepared in the manner of Example 6 using 1.8 g (0.006 mole) of *cis,trans*-3-[2-chloro-3,3,3-trifluoropropenyl]-2,2-dimethylcyclopropanecarbonyl chloride from Example 4, 1.6 g (0.007 mole) of $\alpha$-cyano-3-phenoxybenzyl alcohol, and 0.66 g (0.008 mole) of pyridine in 15 ml of methylene chloride. The pot residue analyzed 99% $\alpha$-cyano-3-phenoxybenzyl *cis,trans*-3-[2-chloro-3,3,3-trifluoropropenyl]-2,2-dimethylcyclopropanecarboxylate, as determined by gas chromatographic analysis. The weight of product was 0.9 g. The nmr and ir spectra were consistent with the assigned structure.

Elemental analysis calc'd for $C_{23}H_{19}ClF_3NO_3$: C 61.46 H 4.26
Found: C 61.47 H 4.48

Insecticidal esters may also be prepared by reacting a diene of the formula

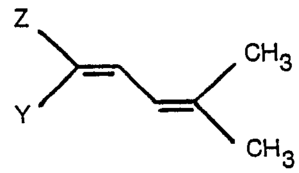

wherein Y and Z are defined above with diazoacetic esters of the formula $N_2CHCO_2R$ where R is lower alkyl or $R^1$ as defined above. The following example is illustrative of this method of preparation.

Example 10
Synthesis of 3-phenoxybenzyl *cis,trans*-3-[2-trifluoromethyl-1-propenyl]-2,2-dimethylcyclopropanecarboxylate

A. Synthesis of (3-methyl-2-butenyl)triphenylphosphonium chloride

A stirred solution of 52.3 g (0.5 mole) of 3-methyl-2-butenyl chloride and 144.1 g (0.55 mole) of triphenylphosphine in 400 ml of toluene was heated at 100°C for 18 hours. The white solid formed was collected by filtration to give 147.6 g of (3-methyl-2-butenyl)triphenylphosphonium chloride.

B. Synthesis of 2-methyl-5-trifluoromethyl-2,4-hexadiene

A stirred solution of 144.3 g (0.39 mole) of (3-methyl-2-butenyl)triphenylphosphonium chloride in 300 ml of methylene chloride, under nitrogen atmosphere, was cooled to 0°C. To this cold solution a solution of 84.2 g (0.39 mole) of sodium methoxide 25% in methanol was added dropwise at a rate to keep the reaction mixture temperature below 4°C. Upon complete addition (40 minutes) a solution of 50.4 g (0.45 mole) of 1,1,1-trifluoroacetone in 5 ml of methylene chloride was added dropwise at a rate to keep the reaction mixture temperature below 6°C. Upon complete addition (40 minutes) the reaction mixture was allowed to warm to ambient temperature and was then stirred for 18 hours. The reaction mixture was washed twice with 150 ml portions of water, then dried with magnesium sulfate. The mixture was filtered and the filtrate concentrated by distillation. Pentane was added to the pot residue to precipitate triphenylphosphine oxide. The mixture was filtered and the filtrate concentrated by distillation. Diglyme was added to the pot residue, and an additional amount of triphenylphosphene oxide was collected by filtration. The filtrate was fractionally distilled by a spinning band distilling system to give in sixteen fractions 14.2 g of 98% 2-methyl-5-trifluoromethyl-2,4-hexadiene; b.p. 122—124°C. The nmr spectrum was consistent with the assigned structure.

C. Synthesis of ethyl *cis,trans*-3-[2-trifluoromethyl-1-propenyl]-2,2-dimethylcyclopropanecarboxylate

To a stirred solution of 11.9 g (0.073 mole) of 2-methyl-5-trifluoromethyl-2,4-hexadiene and 80 ml of rhodium diacetate, prepared in the manner of Mitchell, Rempel, et al., JCS *A*, 3322 (1970), under a nitrogen atmosphere, 8.3 g (0.073 mole) of ethyl diazoacetate was added by means of a syringe. Complete addition required 16.5 hours, due to the vigorous evolution of nitrogen. Upon complete addition the reaction mixture was stirred for 30.5 hours. Gas chromatographic analysis (GC) of the reaction mixture indicated it to contain 44% hexadiene starting material. An additional 6.5 g of ethyl diazoacetate was added dropwise during an 18 hour period. GC analysis of the reaction mixture at the end of 60 hours indicated it to contain 20% hexadiene starting material. The reaction mixture was distilled under reduced pressure [5—12 mm (.67 kPa — 1.6 kPa)] to give 10.2 g of crude product; b.p. 80—85°C/5 mm. (0.67 kPa). The crude product was placed on a chromatographic column of 300 g of silica gel. Elution was accomplished first with pure hexane, then with 3% ethyl acetate in hexane. The chromatography was completed in twenty fractions of 75 or 150 ml each. Appropriate fractions were analyzed by GC and nuclear magnetic resonance (NMR). NMR analysis indicated fraction 9 to be pure *cis, E, Z* isomers, and fractions 10—12 to be pure *cis,trans* isomers. Fractions 9—12 were combined to give 4.3 of ethyl *cis,trans*-3-[2-trifluoromethyl-1-propenyl]-2,2-dimethylcyclopropanecarboxylate.

D. Synthesis of 3-phenoxybenzyl *cis,trans*-3-[2-trifluoromethyl-1-propenyl]-2,2-dimethylcyclopropanecarboxylate

A stirred mixture of 4.2 g (0017 mole) of ethyl *cis,trans*-3-[2-trifluoromethyl-1-propenyl]-2,2-dimethylcyclopropanecarboxylate, 5.0 g (0.025 mole) of 3-phenoxybenzyl alcohol, and 3 drops of titanium isopropylate in 6 ml of nonane, under nitrogen atmosphere, was heated at 140°C for 67

hours in a reaction vessel fitted with short-path distilling head for the removal of the by-product ethanol. GC analysis of the resulting reaction mixture indicated the reaction had gone to near completion. The reaction mixture was placed on a chromatographic column of 300 ml of silica gel in hexane. Elution was accomplished with 1 liter of 3% ethyl acetate in hexane followed by 5% ethyl acetate in hexane. Fractions 1 through 4 were each 500 ml in size. Fraction size was cut to 250 ml in Fractions 5 through 9. Fractions 4 and 5 were combined and distilled using a Kugelrohr distilling system, first at 95°C/0.05 mm (6.7 Pa) to remove low boiling impurities, then at 105°C/0.05 mm (6.7 Pa) to give 5.6 g of 3-phenoxybenzyl *cis,trans*-3-[2-trifluoromethyl-1-propenyl]-2,2-dimethylcyclopropanecarboxylate. The ir and nmr spectra were consistent with the assigned structure.

Analyses calc'd for $C_{23}H_{23}F_3O_3$: C 68.31; H 5.73
Found: C 68.21; H 5.85

The insecticidal compounds of this invention, prepared in accordance with the foregoing examples, are shown in Table IV below.

In the method aspect of this invention an effective insecticidal amount of the compound is applied to the locus where insect control is desired, for example, to the insect itself or to the foliage or seeds of agricultural plants. The compounds are useful for the control of household, veterinary, and crop insects and may be applied as technical material or as a formulated product. Typical formulations include compositions of the active ingredient in combination with an agriculturally acceptable carrier or extender, preferably with a surface-active agent, and optionally with other active ingredients. Suitable formulations include granules, powders, or liquids, the choice varying with the type of pest and environmental factors present at the particular locus of infestation. Thus, the compounds may be formulated as granules of various sizes, as dusts, as wettable powders, as emulsifiable concentrates, as solutions, as dispersions, as controlled release compositions, and the like. A typical formulation may vary widely in concentration of the active ingredient depending upon the particular agent used, the additives and carriers used, other active ingredients and the desired mode of application. With due consideration of these factors, the active ingredient of a typical formulation may, for example, be suitably present at a concentration of about 0.1% up to about 99.5% by weight of the formulation An agriculturally acceptable carrier may comprise about 99.5% by weight to as low as about 0.5% by weight of the formulation. Compatible surface-active agents, if employed in the formulation, may be present at various concentrations, suitably in the range of 1% to 30% by weight of the formulation.

The formulation may be used as such or diluted to a desired use dilution with a diluent or carrier suitable for facilitating dispersion of the active ingredients. The concentration of the active ingredient in the use dilution may be in the range of about 0.01 to about 10% by weight. Many variations of spraying, dusting, and controlled or slow release compositions of a type known in the art may be used by substituting or adding a compound of this invention into the compositions known or apparent to the art.

The compounds of this invention may be formulated and applied with other compatible active agents, including nematicides, insecticides, acaricides, fungicides, plant regulators, herbicides, fertilizers, and the like.

In applying these compounds, whether alone or with other agricultural chemicals, an effective insecticidal amount of the active ingredient must be applied. While the application rate will vary widely depending on the choice of compound, the formulation and mode of application, the plant species being protected, and the planting density, a suitable use rate may be in the range of 0.005 to 3 kg./hectare, preferably 0.01 to about 1 kg./hectare.

The compounds of this invention were tested for initial insecticidal activity as described below.

Example 11

*Initial Contact Activity:* The test compound was dissolved in a small amount of acetone, and the acetone solution was dispersed in water containing one drop of isooctylphenyl polyethoxyethanol to make a solution having 1250 ppm by weight (w/w) or 512 ppm (w/w) active ingredient. Aliquots of this solution were diluted with an appropriate amount of water to provide solutions containing various concentrations of active ingredient. Test organisms and techniques were as follows: the activity against Mexican bean beetle (*Epilachna varivestis* Muls.) and southern armyworm (*Spodoptera eridania* [Cram.]) was evaluated by dipping the leaves of pinto bean plants into the test solution or spraying with the test solution and infesting the leaves with the appropriate immature-form insects after the foliage had dried. The activity against the pea aphid (*Acyrthosiphon pisum* [Harris]) was evaluated on broad bean plants whose leaves were dipped or sprayed before infestation with adult aphids. The activity against twospotted spider mites (*Tetranychus urticae* [Koch]) was evaluated on pinto bean plants whose leaves were dipped or sprayed with test solution after infestation with adult mites. The activity against the milkweed bug (*Oncopeltus faciatus* [Dallas]) and the plum curculio (*Conatrachelus nenuphar* [Herbst]) was evaluated by spraying the test solutions into glass dishes or jars containing the adult insects. Following application of the compound and infestation the tests were maintained in a

holding room at 80°F (26.7°C) and 50% relative humidity for an exposure period of at least 48 hours. At the end of this time the dead and living insects or mites were counted, and the percent kill was calculated. Results of these tests are summarized in Table V. Table V also reports data at 156, 39 and 10 parts per million (ppm) for the commercial insecticide permethrin, 3-phenoxybenzyl (±) *cis,trans*-3-(2,2-dichlorovinyl)-2,2-dimethylcyclopropane-1-carboxylate. The compounds in general exhibit excellent initial activity when compared with that of the reference compound.

Example 12

The compounds of this invention were tested for insecticidal activity by applying to the insect appropriate amounts of a toxicant solution containing 5 mg/l of toxicant in acetone. The tests are read twenty-four hours after application of the toxicant solution and the percent kill determined. The well known commercial insecticide permethrin, 3-phenoxybenzyl (±) *cis,trans*-3-(2,2-dichlorovinyl)-2,2-dimethylcyclopropanecarboxylate, was used as the standard for comparison. Relative potency, based on a value of 1.0 for permethrin was determined by comparing percent kill for the test compound with that for the standard. The insects employed include southern armyworm (*Spodoptera eridania* [Cram.]), cabbage looper (*Trichoplusia ni* [Hubner]), beet armyworm (*Spodoptera exigua* [Hubner]), and corn earworm (*Heliothis zea* [Boddie]), Mexican bean beetle (*Epilachna varivestis* Muls.) and milkweed bug (*Oncopeltus faciatus* [Dallas]).

The results, shown in Table VI, demonstrate the insecticidal compounds of this invention are in general highly toxic against the organisms tested.

The unexpected activity of the insecticides of this invention against aphids is illustrated in the example which follows:

Example 13

In this example the actual aphid activity of certain compounds of the invention was compared with predicted values of these compounds. The predicted values were determined relative to permethrin using the formula:

$$\frac{\text{Lepidopterous Activity Permethrin}}{\text{Lepidopterous Activity Test Compound}} = \frac{\text{Aphid Activity Permethrin}}{\text{Predicted Aphid Activity Test Compound}}$$

This formula was applied to two known compounds (A and B) to predict an expected aphid activity range, and that the actual observed activity for these two compounds is within or very close to the predicted range. Compound A was 3-phenoxybenzyl *cis*-3-(2,2-dichlorovinyl)-2,2-dimethylcyclopropane-1-carboxylate (about 95% *cis* isomer). Compound B was α-cyano-3-phenoxybenzyl 3-(2,2-dichlorovinyl)-2,2-dimethylcyclopropane-1-carboxylate having about 40% *cis* isomer. Permethrin had an observed $LC_{90}$ of 64 ppm, compound A. An $LC_{90}$ of 80 ppm, and compound B in $LC_{90}$ of 50 ppm.

This formula was then applied to certain compounds of this invention. It was found the actual $LC_{90}$ value for compounds tested (compounds 6.02, 6.06 and 6.07) were well below the corresponding values for permethrin, compound A, and compound B. That value for compound 6.02 was also well below the predicted range.

The example which follows illustrates the unexpected stability of certain compounds of this invention to photodegradation in the presence of light and air.

Example 14

An emulsifiable concentrate formulation of the test compound was diluted with toluene to give 220 mg of active ingredient per liter. One hundred μl aliquots were placed in each of six 5.0 cm diameter petri dishes, and the solvent allowed to evaporate leaving a residual deposit of 1.1 μg/cm². Three of the dishes were stored in the dark, and three were exposed to a 275 watt sunlamp at a distance of about 26 cm. After 24 hours, the residual deposit was recovered from each of the six plates, and was analyzed by high performance liquid chromatography for remaining active ingredient. For compound 6.01, 41.6 percent of the starting material was present after 24 hours. Also included are results with permethrin and two additional compounds described by Elliott et al. in U.S. Patent 4,024,163, were included in the test. For permethrin, 19.1% of the starting material was present after 24 hours. For the *cis* isomeric component of permethrin about 21 to 24 percent was present after 24 hours. For the α-cyano analog of the *cis* component of permethrin 20.2 percent remained after 24 hours. The test compound thus demonstrated a two-fold improvement over the reference compounds.

TABLE I

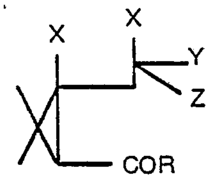

| Example | X | Y | Z | R |
|---|---|---|---|---|
| 1.1[a] | Br | $CF_3$ | Br | $OCH_3$ |
| 1.2[a] | Cl | $CF_3$ | F | $OCH_3$ |
| 1.3[a] | Cl | $CF_3$ | H | $OCH_3$ |
| 1.4[a] | Cl | $CF_2Cl$ | Cl | $OCH_3$ |
| 1.5[a] | Cl | $CF_2Cl$ | F | $OCH_3$ |
| 1.6[a] | Cl | $CFCl_2$ | F | $OCH_3$ |
| 1.7[a] | Cl | $CF_2CF_2Cl$ | Cl | $OCH_3$ |
| 1.8[b] | Cl | $C_2F_5$ | Cl | $OCH_3$ |

a) Boiling points in °C/mmHg (Pa or kPa): 1.1: 63/0.08 (10.7 Pa); 1.2:71/0.09 mm (12 Pa); 1.3: 112—115°/7 (.93 kPa); 1.4: 95—106/0.1—0.125 (13.3—16.6 Pa); 1.5: 58—60/0.005 (0.67 Pa); 1.6: 103/0.2—0.3 (26.6—40 Pa); 1.7: 98—102(0.05 (6.7 Pa).
b) Structure confirmed by nmr.

TABLE II

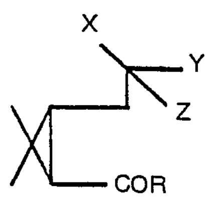

| Example | X | Y | Z | R |
|---|---|---|---|---|
| 2.1[a] | Br | $CF_3$ | Br | $OCH_3$ |
| 2.2 | Cl | $CF_3$ | F | $OCH_3$ |
| 2.3[b] | Cl | $CF_3$ | H | $OCH_3$ |
| 2.4 | Cl | $CF_2Cl$ | Cl | $OCH_3$ |
| 2.5[a] | Cl | $CF_2Cl$ | F | $OCH_3$ |
| 2.6 | Cl | $CFCl_2$ | F | $OCH_3$ |
| 2.7[b] | Cl | $CF_2CF_2Cl$ | Cl | $OCH_3$ |

a) Boiling points: in °C/mm Hg (Pa or kPa): 2.1: 100—113°/0.09—0.1 (12—13.3 Pa); 2.5: 45—47°/0.02 (2.67 Pa).
b) Structure confirmed by nmr.

TABLE III

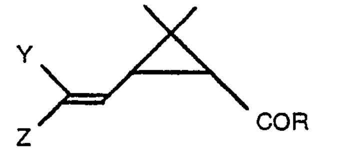

| Example | Y | Z | R | Isomer |
|---|---|---|---|---|
| 3.1[b] | $CF_3$ | Br | $OCH_3$ | c/t |
| 3.2[b] | $CF_3$ | F | $OCH_3$ | c/t |
| 3.3 | $CF_3$ | H | $OCH_3$ | c/t |
| 3.4[b] | $CF_2Cl$ | Cl | $OCH_3$ | c/t |
| 3.5[b] | $CF_2Cl$ | F | $OCH_3$ | c/t |
| 3.6[b] | $CFCl_2$ | F | $OCH_3$ | c/t |
| 3.7[b] | $CF_2CF_2Cl$ | Cl | $OCH_3$ | c/t |
| 3.8[b] | $C_2F_5$ | Cl | $OCH_3$ | c/t |
| 4.1[c] | $CF_3$ | Br | OH | c/t |
| 4.2[d] | $CF_3$ | F | OH | c/t |
| 4.3[a] | $CF_3$ | H | OH | c/t |
| 4.4[a] | $CF_2Cl$ | Cl | OH | c/t |
| 4.5[c] | $CF_2Cl$ | F | OH | c |
| 4.6[c] | $CF_2CF_2Cl$ | Cl | OH | c/t |
| 4.7 | $C_2F_5$ | Cl | OH | c/t |
| 5.1[e] | $CF_3$ | Br | Cl | c/t |
| 5.2[e] | $CF_3$ | F | Cl | c/t |
| 5.3[f] | $CF_3$ | H | Cl | c/t |
| 5.4[e] | $CF_2Cl$ | Cl | Cl | c/t |
| 5.5[d,e] | $CF_2Cl$ | F | Cl | c |
| 5.6[d,e] | $CF_2CF_2Cl$ | Cl | Cl | c/t, *Z* |
| 5.7[b] | $C_2F_5$ | Cl | Cl | c/t, *Z* |
| 5.8[d,e] | $CF_3$ | Cl | Cl | c/t, *E*, *Z* |

a NMR spectrum consistent with assigned structure.
b Boiling points in °C/mm Hg (Pa or kPa): 3.1: 44—47°/0.07—0.08 (9.3—10.6 Pa); 3.2: 71°/29 (3.9 kPa); 3.4: 84—88°/1.25—1.4 (0.17—0.19 kPa); 3.5: 90—92°/11 (1.5 kPa); 3.6: 60—71°/0.08 (10.7 Pa); 3.7: 59—65°/0.07 (9.3 Pa); 3.9: 98—110°/7 (0.93 kPa); 5.7: 42—51°/0.1 (13.3 Pa);
c Melting points (°C): 4.1:110—116°; 4.5:80—87°; 4.6:67—69°
d Structure confirmed by IR spectra
e Liquid, not isolated
f Semi-solid, not isolated

TABLE IV

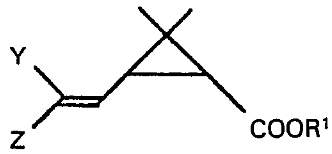

| Compound[a] | Y | Z | R[1] | Isomer |
|---|---|---|---|---|
| 6.01 | $CF_3$ | Cl | 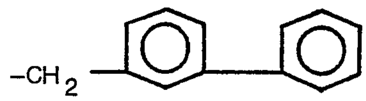 | c |
| 6.02 | $CF_3$ | Br | " | c |
| 6.03 | $CF_3$ | Br | " | t |
| 6.04 | $CF_3$ | F | " | c |
| 6.05 | $CF_3$ | F | " | t |
| 6.06 | $CF_3$ | Cl | 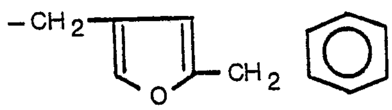 | c |
| 6.07 | $CF_3$ | Cl | " | c/t |
| 6.12[b] | $CF_2Cl$ | Cl | 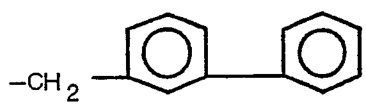 | c |
| 6.13[b] | $CF_2Cl$ | F | " | c |
| 6.14 | $C_2F_5$ | Cl | " | c |
| 6.15[d] | $CF_2CF_2Cl$ | Cl | " | c |
| 6.16 | $CF_3$ | H | " | c |
| 6.17 | $CF_3$ | Cl | 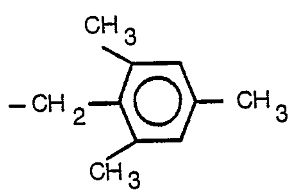 | c/t |
| 6.18 | $CF_3$ | Cl | 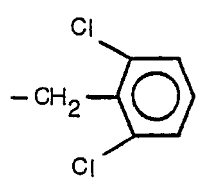 | t |

TABLE IV (Continued)

| Compound[a] | Y | Z | $R^1$ | Isomer |
|---|---|---|---|---|
| 6.19 | $CF_3$ | Cl | CH $CF_3$ | c/t |
| 6.20 | $CF_3$ | Cl | $CH_2$ $C(CH_3)_3$ | c/t |
| 6.21 | $CF_3$ | Cl | 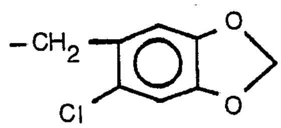 | c/t |
| 6.22 | $CF_3$ | Cl | 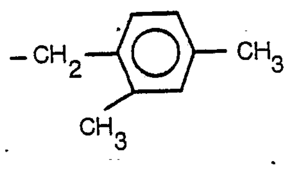 | c/t |
| 6.23 | $CF_3$ | Cl | 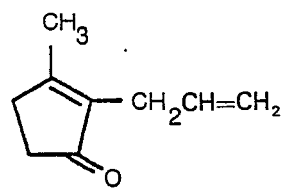 | c/t |
| 6.24 | $CF_3$ | Cl | 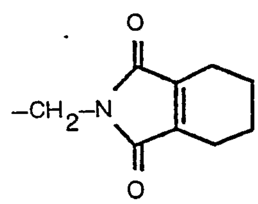 | c/t |
| 6.25 | $CF_3$ | Cl | 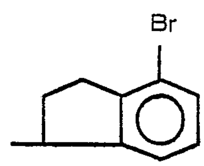 | c |
| 6.26 | $CF_3$ | Cl | 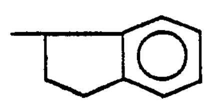 | c |
| 6.27 | $CF_3$ | Cl | 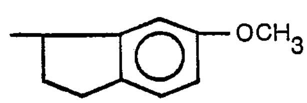 | c |
| 6.28 | $CF_3$ | Cl | 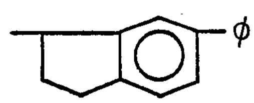 | c |

TABLE IV (Continued)

| Compound[a] | Y | Z | R¹ | Isomer |
|---|---|---|---|---|
| 6.29 | $CF_3$ | Cl | 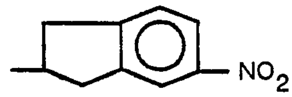 | c |
| 6.30 | $CF_3$ | Cl | 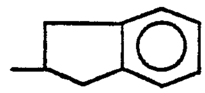 | c |

* Separated diostereoisomers
a Assigned structure confirmed by nmr and elemental analysis
b B.P. 100—102°C/0.005 mm Hg (0.67 Pa)
c B.P. 110°C/0.9 mm Hg (.12 Pa)
d NMR analysis only

TABLE V

INITIAL ACTIVITY

| COMPOUND[a] | CONC.[b] | PERCENT KILL MWB[1] | MBB[2] | SAW[3] | PA[4] | SM[5] | PC[6] |
|---|---|---|---|---|---|---|---|
| 6.01 | 20 (39) | 10 | 100 | 100 | 100 | (50) | 10 |
| 6.02 | 64 | — | 100 | 100 | 100 | 0 | — |
| 6.03 | 64 (512) | — | 95 | 100 | 71 | (0) | — |
| 6.04 | 78 (5) | 41 | (100) | (100) | 100 | 100 | 55 |
| 6.06 | 64 | — | 100 | 100 | 100 | 0 | — |
| 6.07 | 64 | — | 100 | 100 | 100 | 0 | — |
| 6.13 | 78 | 95 | 100 | 100 | 100 | 94 | 25 |
| 6.14 | 78 | 25 | 100 | 100 | 100 | 80 | 0 |
| 6.15 | 78 (312) | 10 | 100 | 100 | 90 | 58 | (0) |
| 6.16 | 78 | 55 | 100 | 100 | 90 | 92 | 0 |
| 6.17 | 20 (312) | 5 | 100 | 100 | 92 | (31) | — |
| 6.18 | 20 (312) | 10 | 95 | 100 | 100 | 19 | — |
| 6.19 | 78 | 5 | 100 | 100 | 100 | — | 5 |
| 6.20 | 39 | 10 | 100 | 0 | 0 | 0 | 0 |
| 6.21 | 64 | — | 65 | 100 | 65 | 0 | — |
| 6.22 | 64 | — | 45 | 0 | 0 | 0 | — |
| 6.23 | 64 | — | 45 | 20 | 0 | 0 | — |
| 6.24 | 64 | — | 100 | 30 | 0 | 0 | — |
| 6.26 | 512 | — | 100 | 100 | 43 | 0 | — |
| 6.27 | 512 | — | 85 | 100 | 100 | 0 | — |
| 6.28 | 512 | — | 100 | 100 | 100 | 0 | — |
| 6.29 | 64 | — | 100 | 100 | 50 | 65 | — |
| 6.30 | 64 | — | 80 | 0 | 20 | 0 | — |
| permethrin | 156 | 71 | — | — | 94 | 36 | 100 |
| | 39 | 30 | 100 | 100 | 93 | — | 33 |
| | 10 | 10 | 75 | 100 | 54 | — | 15 |
| CHECK[d] | — | 0 | 0 | 5 | 10 | 0 | 0 |

a. Structure in Table IV
b. Concentration in parts per million
c. Data in parenthesis taken at concentration shown in parenthesis
d. Untreated sample

1. *Oncopeltus faciatus* [Dallas]
2. *Epilachna varivestis* Muls.
3. *Spodoptera eridania* [Cram.]
4. *Acyrthosiphon pisum* [Harris]
5. *Tetranychus urticae* [Koch]
6. *Conatrachelus nenuphar* [Herbst]

TABLE VI

| Compound* | Relative Potency Against SAW[1] | CL[2] | BAW[3] | CEW[4] | MBB[5] | MWB[6] |
|---|---|---|---|---|---|---|
| 6.01 | 1.0 | 0.8 | 1.6 | 0.7 | 0.7 | 3.6 |
| 6.02 | 1.8 | 1.7 | 2.8 | — | 1.6 | 6.6 |
| 6.03 | 0.1 | 0.06 | — | — | — | — |
| 6.04 | 0.9 | 0.5 | — | — | — | — |
| 6.05 | 0.08 | <0.001 | — | — | — | — |
| 6.06 | 12.2 | 11.8 | 24.1 | — | 7.9 | 67.6 |
| 6.07 | 6.4 | 8.2 | 7.9 | | 3.8 | 55.7 |
| 6.12 | 1.3—1.6 | 0.9 | — | — | — | — |
| 6.13 | 0.4 | 0.1 | — | — | — | — |
| 6.14 | 0.03 | <0.001 | — | — | — | — |
| 6.15 | 0.01 | <0.001 | — | — | — | — |
| 6.16 | 0.1 | <0.001 | — | — | — | — |
| 6.17 | 0.08 | 0.09 | — | — | — | — |
| 6.18 | 0.2 | 0.05 | — | — | — | — |
| 6.19 | 0.06 | 0.05 | — | — | — | — |
| 6.20 | <0.001 | <0.001 | — | — | — | — |
| 6.21 | 0.06 | — | — | — | 0.06 | 0.6 |
| 6.22 | 0.05 | — | — | — | 0.01 | 0.4 |
| 6.23 | 0.01 | — | — | — | 1.0 | 0.07 |
| 6.24 | 0.04 | — | — | — | 0.5 | <0.001 |
| 6.25 | 0.05 | 0.06 | — | — | — | — |
| 6.26 | 0.03 | 0.04 | — | — | — | — |
| 6.28 | 0.01 | — | — | — | — | — |
| 6.29 | 0.4 | 0.2 | — | — | — | — |
| 6.30 | 0.2 | 0.2 | — | — | — | — |

1. *Spodoptera eridania* [Cram.]
2. *Trichoplusia ni* [Hubner]
3. *Spodoptera exigua* [Hubner]
4. *Heliothis zea* [Boddie]
5. *Epilachna varivestis* Muls.
6. *Oncopeltus faciatus* [Dallas]

* See Table IV for structure

**Claims**

1. Perhaloalkylvinylcyclopropanecarboxylates of the formula

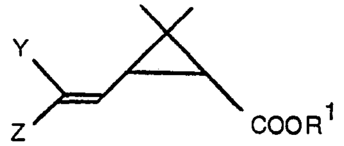

(I)

characterized in that one of Y and Z is a perhaloalkyl group having 1 to 4 carbon atoms and the other is hydrogen, halogen, lower alkyl, phenyl, phenylthio or benzyl, or Y and Z together form a perhalocyclopentylidine group; and $R^1$ is allethrolonyl, tetrahydrophthalimidomethyl, or is an alcohol residue represented by the formula

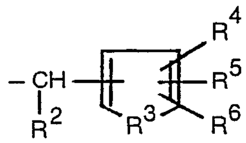

or

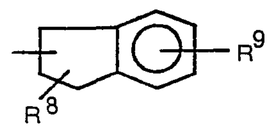

wherein $R^2$ is hydrogen, lower alkyl, ethynyl, cyano, or trihalomethyl; $R^3$ is divalent oxygen, divalent sulfur, or vinylene; $R^4$, $R^5$ and $R^6$ are independently hydrogen, lower alkyl, halogen, lower alkenyl, phenyl, phenoxy, benzyl, phenylthio, or any two of $R^4$, $R^5$ and $R^6$ are joined to form a divalent methylenedioxy group attached to two adjacent ring carbon atoms of a phenyl ring, and when $R^4$, $R^5$ or $R^6$ contains a phenyl ring that phenyl ring may be substituted with one to three substituents selected from halogen and lower alkyl; $R^8$ is hydrogen, lower alkyl, lower alkoxy, lower alkenyl, lower alkenyloxy, phenyl or benzyl; $R^9$ is hydrogen, halogen, lower alkyl, lower alkoxy, lower alkenyl, lower alkenyloxy, lower alkylthio, lower alkylsulfinyl, lower alkylsulfonyl, phenyl, phenoxy, benzoyl, nitro, or cyano, with the proviso that when one of Y and Z is a perhaloalkyl group, the other of Y and Z is halogen or methyl, $R^3$ is vinylene, and two of $R^4$, $R^5$ and $R^6$ are hydrogen, then

(a) the other of $R^4$, $R^5$ and $R^6$ is not phenoxy, or

(b) $R^2$ is not hydrogen, ethynyl or cyano.

2. A compound of claim 1 characterized in that $R^2$ and $R^3$ have the same meaning as in claim 1, $R^6$ is hydrogen, $R^4$ and $R^5$ are as defined in claim 1 but are other than halogen, with the proviso that when $R^4$ or $R^5$ contains a phenyl ring that phenyl ring is not substituted.

3. A compound of claim 1 characterized in that $R^1$ represents the alcohol residue in which $R^3$ is oxygen.

4. A compound of claims 2 and 3 characterized in that $R^1$ is 5-benzyl-3-furylmethyl.

5. A compound of claim 1 characterized in that $R^1$ represents the alcohol residue in which $R^3$ is vinylene, $R^4$ is optionally substituted phenyl and $R^5$ and $R^6$ are each other than optionally substituted phenyl.

6. A compound of claim 5 characterized in that $R^1$ is optionally substituted 3-phenylbenzyl.

7. A compound of claim 1 characterized in that $R^1$ represents the alcohol residue

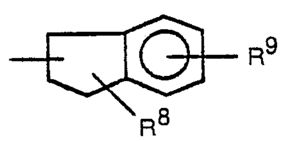

wherein $R^8$ and $R^9$ are as defined above.

8. A compound of claim 7, characterized in that $R^1$ represents the alcohol residue

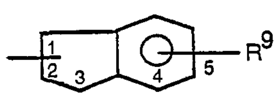

in which $R^9$ is phenyl.

9. The compound of claim 7 characterized in that $R^1$ is

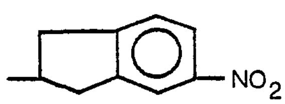

10. A compound according to each of claims 1 through 9, characterized in that one of Y and Z is trihalomethyl.

11. A compound according to each of claims 1 through 10, characterized in that one of Y and Z is trifluoromethyl and the other is halogen.

12. A process for preparing the compound of claims 1 through 11, characterized by dehydrohalogenating an alkanoate of the formula

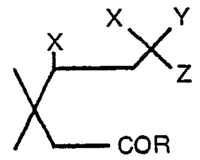

(II)

wherein X is chloro or bromo, Y and Z are as defined in claims 1, 9 or 10, and R is lower alkoxy having 1 to 4 carbons, to form a compound of the formula

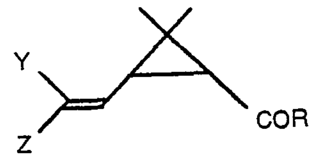

(III)

wherein Y, Z and R are as defined above, then converting the compound of formula III to a compound of the formula I

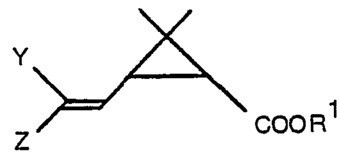

(I)

wherein $R^1$ is as defined in claims 1 through 9 and Y and Z are as defined above.

13. An insecticidal composition, characterized in containing an insecticidal amount of the compound of any of claims 1 through 11 in admixture with a compatible agriculturally acceptable carrier or adjuvant.

14. A method for insect control, characterized in applying to the locus where control is desired an insecticidally effective amount of the compound of any of claims 1 through 11.

**Patentansprüche**

1. Perhalogenalkylvinylcyclopropancarboxylate der Formel

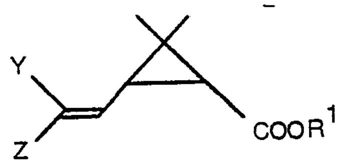

(I)

dadurch gekennzeichnet, daß ein Rest aus der Gruppe Y und Z ein Perhalogenalkylrest mit 1 bis 4 Kohlenstoffatomen ist und der andere Rest ein Wasserstoffatom, Halogenatom, eine niedere Alkylgruppe, einen Phenyl-, Phenylthio- oder einen Benzylrest bedeutet, oder Y und Z zusammengenommen eine Perhalogencyclopentylidingruppe darstellen; und $R^1$ einen Allethrolonyl- oder Tetrahydrophthalimidomethylrest oder einen Alkoholrest der nachstehenden allgemeinen Formel bedeutet:

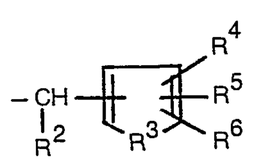

oder

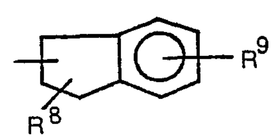

(II)

wobei in den Formeln $R^2$ ein Wasserstoffatom, einen niederen Alkylrest, Äthinylrest, Cyangruppe oder einen Trihalogenmethylrest bedeutet, $R^3$ einen zweiwertigen Sauerstoffrest, zweiwertigen Schwefelrest oder eine Vinylengruppe, $R^4$, $R^5$ und $R^6$ unabhängig voneinander einen Rest aus der Gruppe Wasserstoffatom, Niederalkylrest, Halogenatom, Niederalkenylrest, Phenylgruppe, Phenoxygruppe, Benzylrest und Phenylthiogruppe bedeuten, oder jeweils zwei Reste aus der Gruppe $R^4$, $R^5$ und $R^6$ verbunden sind und eine zweiwertige Methylendioxygruppe bilden, die mit zwei benachbarten Ringkohlenstoffatomen eines

Phenylrings verknüpft ist, und sofern die Reste $R^4$, $R^5$ oder $R^6$ einen Phenylring enthalten, so kann dieser mit 1 bis 3 Substituenten aus der Gruppe Halogenatom und Niederalkylrest substituiert sein; $R^8$ ein Wasserstoffatom, eine Niederalkyl-, Niederalkoxy-, Niederalkenyl-, Niederalkenyloxy-, Phenyl- oder Benzylgruppe bedeutet; $R^9$ ein Wasserstoffatom, Halogenatom, einen Niederalkyl-, Niederalkoxy-, Niederalkenyl-, Niederalkenyloxy-, Niederalkylthio-, Niederalkylsulfinyl-, Niederalkylsulfonyl-, Phenyl-, Phenoxy- oder Benzoylrest oder eine Nitro- oder Cyangruppe bedeutet, mit der Maßgabe, daß, wenn ein Rest aus der Gruppe Y und Z einen Perhalogenalkylrest bedeutet, der andere Rest aus Der Gruppe Y und Z ein Halogenatom oder eine Methylgruppe bedeutet, $R^3$ eine Vinylengruppe darstellt und zwei Reste aus der Gruppe $R^4$, $R^5$ und $R^6$ Wasserstoffatome sind,

(a) der weitere Rest aus der Gruppe $R^4$, $R^5$ und $R^6$ keine Phenoxygruppe bedeutet, oder

(b) $R^2$ nicht ein Wasserstoffatom, oder Äthinylgruppe oder eine Cyangruppe darstellt.

2. Verbindung nach Anspruch 1, dadurch gekennzeichnet, daß $R^2$ und $R^3$ die in Anspruch 1 angegebene Bedeutung haben, $R^6$ ein Wasserstoffatom bedeutet, $R^4$ und $R^5$ die in Anspruch 1 angegebene Bedeutung haben, jedoch nicht ein Halogenatom darstellen, mit der Maßgabe, daß, wenn $R^4$ oder $R^5$ einen Phenylring enthalten, dieser Phenylring unsubstituiert ist.

3. Verbindung nach Anspruch 1, dadurch gekennzeichnet, daß $R^1$ einen Alkoholrest bedeutet, in dem $R^3$ ein Wasserstoffatom darstellt.

4. Verbindung nach den Ansprüchen 2 und 3, dadurch gekennzeichnet, daß $R^1$ einen 5-Benzyl-3-furylmethylrest bedeutet.

5. Verbindung nach Anspruch 1, dadurch gekennzeichnet, daß $R^1$ einen Alkoholrest bedeutet, in dem $R^3$ eine Vinylengruppe, $R^4$ eine gegebenenfalls substituierte Phenylgruppe und $R^5$ und $R^6$ jeweils keinen gegebenenfalls substituierten Phenylrest bedeutet.

6. Verbindung nach Anspruch 5, dadurch gekennzeichnet, daß $R^1$ einen gegebenenfalls substituierten 3-Phenylbenzylrest bedeutet.

7. Verbindung nach Anspruch 1, dadurch gekennzeichnet, daß $R^1$ den Alkoholrest

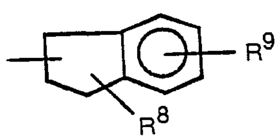

bedeutet, in dem $R^8$ und $R^9$ die vorstehende Bedeutung haben.

8. Verbindung nach Anspruch 7, dadurch gekennzeichnet, daß $R^1$ einen Alkoholrest

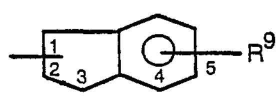

bedeutet, in dem $R^9$ eine Phenylgruppe bedeutet.

9. Verbindung nach Anspruch 7, dadurch gekennzeichnet, daß $R^1$ die Gruppe

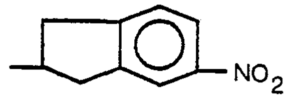

bedeutet.

10. Eine Verbindung nach jedem der Ansprüche 1 bis 9, dadurch gekennzeichnet, daß einer der Reste Y und Z eine Trihalogenmethylgruppe bedeutet.

11. Eine Verbindung nach jedem der Ansprüche 1 bis 10, dadurch gekennzeichnet, daß einer der Reste Y und Z eine Trifluoromethylgruppe bedeutet und der andere Rest ein Halogenatom darstellt.

12. Verfahren zur Herstellung von Verbindungen nach den Ansprüchen 1 bis 11, dadurch gekennzeichnet, daß man ein Alkanoat der Formel

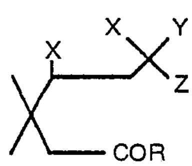

(II)

dehydrohalogeniert, wobei X ein Chlor- oder Bromatom, Y und Z die in den Ansprüchen 1, 9 oder 10 angegebene Bedeutung haben und R einen Niederalkoxyrest mit 1 bis 4 Kohlenstoffatomen bedeutet, wobei man eine Verbindung der Formel III erhält

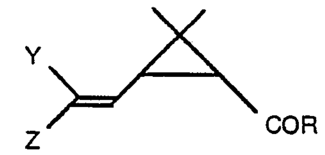

(III)

wobei in der Formel die Reste Y, Z und R die vorstehende Bedeutung haben, und man die Verbindung der Formel III in eine Verbindung der Formel I umwandelt

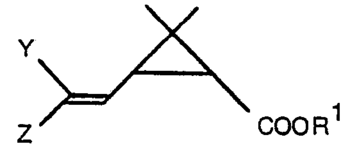

(I)

in der $R^1$ die in den Ansprüchen 1 bis 9 angegebene Bedeutung hat und Y und Z die vorstehende Bedeutung haben.

13. Insektizide Zusammensetzung, gekennzeichnet, durch einen insektizid wirksamen Gehalt einer Verbindung nach jedem der Ansprüche 1 bis 11, im Gemisch mit einem kompatiblen, landwirtschaftlich verträglichen Trägerstoff oder Hilfsmittel.

14. Verfahren zur Bekämpfung von Insekten, dadurch gekennzeichnet, daß man an der Stelle, an der die Bekämpfung gewünscht wird, eine insektizid wirksame Menge der Verbindung nach jedem der Ansprüche 1 bis 11 anwendet.

**Revendications**

1. Perhaloalcoylvinylcyclopropanecarboxylates de formule

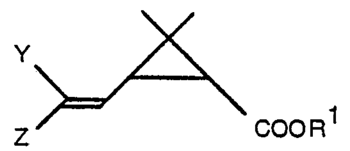

(I)

caractérisés en ce qu'un de Y et Z est un groupe perhaloalcoyle ayant 1 à 4 atomes de carbone et l'autre est un hydrogène, un halogène, un alcoyle inférieur, un phényle, un phénylthio ou un benzyle, ou Y et Z forment ensemble un groupe perhalocyclopentylidine; et $R^1$ est un alléthrolonyle, un tétrahydrophtalimidométhyle ou un reste d'alcool représenté par la formule

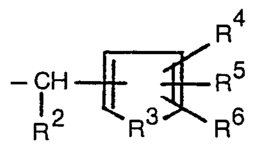

ou

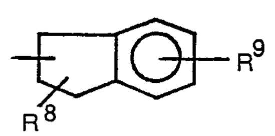

dans laquelle $R^2$ est un hydrogène, un alcoyle, inférieur, un éthynyle, un cyano ou un trihalométhyle; $R^3$ est un oxygène divalent, un soufre divalent ou un vinylène, $R^4$, $R^5$ et $R^6$ sont indépendamment l'un de l'autre un hydrogène, un alcoyle inférieur, un halogène, un alcényle inférieur, un phényle, un phénoxy, un benzyle, un phénylthio, ou deux quelconques des radicaux $R^4$, $R^5$ et $R^6$ sont unis pour former un groupe méthylènedioxy divalent fixé à 2 atomes de carbone cycliques adjacents d'un cycle phényle, et lorsque $R^4$, $R^5$ et $R^6$ contient un cycle phényle, ce cycle phényle peut être substitué par 1 à 3 substituants choisis parmi un halogène et un alcoyle inférieur; $R^8$ est un hydrogène, un alcoyle inférieur, un alcoxy inférieur, un alcényle inférieur, un alcényloxy inférieur, un phényle ou un benzyle; $R^9$ est un hydrogène, un halogène, un alcoyle inférieur, un alcoxy inférieur, un alcényle inférieur, un alcényloxy inférieur, un alcoylthio inférieur, un alcoylsulfinyle inférieur, un alcoylsulfonyle inférieur, un phényle, un phénoxy, un benzoyle, un nitro ou un cyano, sous réserve que lorsqu'un de Y et Z est un groupe perhaloalcoyle, l'autre de Y et de Z est un halogène ou un méthyle, $R^3$ est un vinylène et deux des radicaux de $R^4$, $R^5$ et $R^6$ sont un hydrogène, et

(a) l'autre des radicaux $R^4$, $R^5$ et $R^6$ n'est pas un phénoxy, ou

(b) $R^2$ n'est pas un hydrogène, un éthynyle ni un cyano.

2. Composé selon la revendication 1, caractérisé en ce que $R^2$ et $R^3$ ont la même signification que dans la revendication 1, $R^6$ est un hydrogène, $R^4$ et $R^5$ sont comme défini dans la revendication 1 mais

sont autre qu'un halogène sous réserve que lorsque $R^4$ ou $R^5$ contient un cycle phényle, ce cycle phényle n'est pas substitué.

3. Composé selon la revendication 1, caractérisé en ce que $R^1$ représente le reste d'alcool dans lequel $R^3$ est un oxygène.

4. Composé selon les revendications 2 et 3, caractérisé en ce que $R^1$ est un 5-benzyl-3-furylméthyle.

5. Composé selon la revendication 1, caractérisé en ce que $R^1$ représente le reste d'alcool dans lequel $R^3$ est un vinylène, $R^4$ est un phényle éventuellement substitué et $R^5$ et $R^6$ sont chacun autre qu'un phényle eventuellement substitué.

6. Composé selon la revendication 5, caractérisé en ce que $R^1$ est un 3-phénylbenzyl éventuellement substitué.

7. Composé selon la revendication 1, caractérisé en ce que $R^1$ représente le reste d'alcool

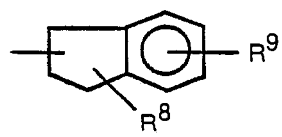

où $R^8$ et $R^9$ sont comme défini ci-dessus.

8. Composé selon la revendication 7, caractérisé en ce que $R^1$ représente le reste d'alcool

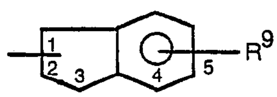

où $R^9$ est un phényle.

9. Composé selon la revendication 7, caractérisé en ce que $R^1$ est

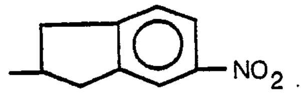

10. Composé selon chacune des revendications 1 à 9, caractérisé en ce qu'un de Y et Z est un trihalométhyle.

11. Composé selon l'une quelconque des revendications 1 à 10, caractérisé en ce qu'un de Y et Z est un trifluorométhyle et l'autre est un halogène.

12. Procédé pour préparer le composé des revendications 1 à 11, caractérisé par la déshydrohalogénation d'un alcanoate de formule

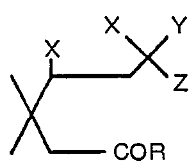

dans laquelle X est un chloro ou bromo, Y et Z sont comme défini dans les revendications 1, 9 ou 10 et R est alcoxy inférieur ayant 1 à 4 carbones, pour former un composé de formule

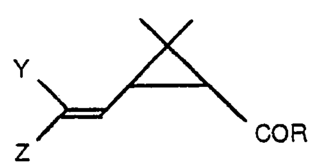

(III)

dans laquelle Y, Z et R sont comme défini ci-dessus, puis la conversion du composé de formule III en un composé de formule I

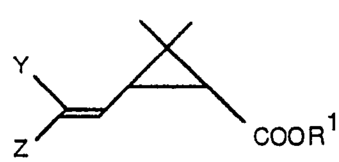

(I)

dans laquelle $R^1$ est comme défini selon les revendications 1 à 9 et Y et Z sont comme défini ci-dessus.

13. Composition insecticide caractérisée en ce qu'elle contient une quantité insecticide du composé de l'une quelconque des revendications 1 à 11 en mélange avec un support ou adjuvant compatibles acceptables en agriculture.

14. Procédé de lutte contre les insectes, caractérisé en ce que l'on applique au site où on désire effectuer la lutte une quantité d'insecticide efficace du composé selon l'une quelconque des revendications 1 à 11.